# EUROPEAN PATENT SPECIFICATION

(11) **EP 2 216 412 B1**
(45) Date of publication and mention of the grant of the patent: **03.10.2012**
(21) Application number: 10005542.5
(22) Date of filing: 06.07.2007
(51) Int. Cl.: C12P 19/26, C08B 37/00

(54) **Process for purification of high molecular weight hyaluronic acid**
Verfahren zur Reinigung von Hyaluronsäure mit hohem Molekulargewicht
Procédé de purification d'acide hyaluronique a poids moleculaire élevé

(30) Priority: 06.07.2006 IN MU10652006; 13.11.2006 IN MU18742006
(43) Date of publication of application: 11.08.2010
(62) Divisional of application: 07859586.5
(73) Proprietor: Reliance Life Sciences Pvt., Ltd., Mumbai 400 701 Maharashtra (IN)
(72) Inventor: Santosh, Yvas, Navi Mumbai 400 701 Maharashtra (IN); Dharmendra, Jain, Navi Mumbai 400 701 Maharashtra (IN); Nataraj, Vedapuri, Navi Mumbai 400 701 Maharashtra (IN); Velankar, Harshad, Navi Mumbai 400 701 Maharashtra (IN); Kapat, Arnub, Navi Mumbai 400 701 Maharashtra (IN); Rangaswamy, Vidhya, Navi Mumbai 400 701 Maharashtra (IN)
(74) Representative: Srinivasan, Ravi Chandran

(56) References cited:
- WO-A-00/44925
- WO-A-86/04355
- WO-A-92/08799
- US-A- 4 897 349
- DATABASE WPI Week 199741 Thomson Scientific, London, GB; AN 1997-447031 XP002474230 & RU 2 074 863 C1 (NART RES PRODN FIRM) 10 March 1997 (1997-03-10)
- ARMSTRONG D C ET AL: "Culture conditions affect the molecular weight properties of hyaluronic acid produced by Streptococcus zooepidermicus" APPLIED AND ENVIRONMENTAL MICROBIOLOGY, WASHINGTON,DC, US, vol. 63, 1997, pages 2759-2764, XP002436439 ISSN: 0099-2240
- JIANFA ZHANG ET AL: "A serum-free medium for colony growth and hyaluronic acid production by Streptococcus zooepidemicus NJUST01" APPLIED MICROBIOLOGY AND BIOTECHNOLOGY, SPRINGER-VERLAG, BE, vol. 72, no. 1, 13 December 2005 (2005-12-13), pages 168-172, XP019421985 ISSN: 1432-0614
- DATABASE WPI Week 198805 Thomson Scientific, London, GB; AN 1988-031296 XP002474231 & JP 62 289198 A (NIPPON KAYAKU KK) 16 December 1987 (1987-12-16)
- BLANK LARS M ET AL: "Stable production of hyaluronic acid in Streptococcus zooppidemicus chemostats operated at high dilution rate" BIOTECHNOLOGY AND BIOENGINEERING, WILEY & SONS, HOBOKEN, NJ, US, vol. 90, no. 6, June 2005 (2005-06), pages 685-693, XP002464474 ISSN: 0006-3592
- RANGASWAMY VIDHYA ET AL: "An efficient process for production and purification of hyaluronic acid from Streptococcus equi subsp zooepidemicus" BIOTECHNOLOGY LETTERS, vol. 30, no. 3, March 2008 (2008-03), pages 493-496, XP002474229 ISSN: 0141-5492

## Description

### CROSS-REFERENCES TO RELATED APPLICATIONS

This application claims benefit of provisional Indian Application No. 1065/MUM/2006 filed on July 6, 2006 and 1874/MUM/2006, filed on November 13, 2006.

### FIELD OF THE INVENTION:

The present invention relates to improved techniques for purifying hyaluronic acid and its salt. The present invention likewise relates to the purification of hyaluronic acid and its salt, having biomedical applications.

### BACKGROUND OF THE INVENTION

Hyaluronic acid (HA) is a naturally occurring biopolymer having, biological functions in bacteria and higher animals including humans. Naturally occurring HA may be found in the tissue of higher animals, in particular as intercellular space filler. (Balazs, Viscosupplementation: a new concept in the treatment of osteoarthritis. J. Rheumatol. Suppl.; 39:3-9, (Aug. 1993)). It is found in greatest concentrations in the vitreous humor of the eye and in the synovial fluid of articular joints. (O'Regan, M., Martini, L, Crescenzi, F., De Luca, C., and Lansing, M., Molecular mechanisms and genetics of hyaluronan biosynthesis. International Journal of Biological Macromolecules 16: 283-286 (1994)). In gram-positive *Streptococci,* it is found as a mucoid capsule surrounding the bacterium.

Hyaluronic acid, also called hyaluronan or hyaluronate, is a glycosaminoglycan distributed widely throughout connective, epithelial, and neural tissues. It is one of the chief components of the extracellular matrix. It contributes significantly to cell proliferation and migration, and may also be involved in the progression of some malignant tumors. Hyaluronan is naturally found in many tissues of the body such as skin, cartilage and the vitreous humor. It is therefore well suited to biomedical applications targeting these tissues. The first hyaluronan biomedical product, Healon®, was developed in the 1970s and 1980s, and was approved for use in eye surgery (i.e., corneal transplantation, cataract surgery, glaucoma surgery and surgery to repair retinal detachment). Other biomedical companies also produce brands of hyaluronan for ophthalmic surgery.

Hyaluronan is also used to treat osteoarthritis of the knee. Such treatments are administered as a course of injections into the knee joint and may act to supplement the viscosity of the joint fluid thereby lubricating the joint, cushioning the joint and producing an analgesic effect. Hyaluronan also may have positive biochemical effects on cartilage cells.

Due to its high biocompatibility and its common presence in the extracellular matrix of tissues, hyaluronan is gaining popularity as a biomaterial scaffold in tissue engineering research. In some cancers, hyaluronan levels correlate well with malignancy and poor prognosis. Hyaluronan may be used as a tumor marker for prostate and breast cancer. It may also be used to monitor the progression of these diseases.

Hyaluronan may also be used postoperatively to induce tissue healing, notably after cataract surgery. Current models of wound healing propose that larger polymers of hyaluronic acid appear in the early stages of healing to physically make room for white blood cells which mediate the immune response. Hyaluronan is a common ingredient in skin care products.

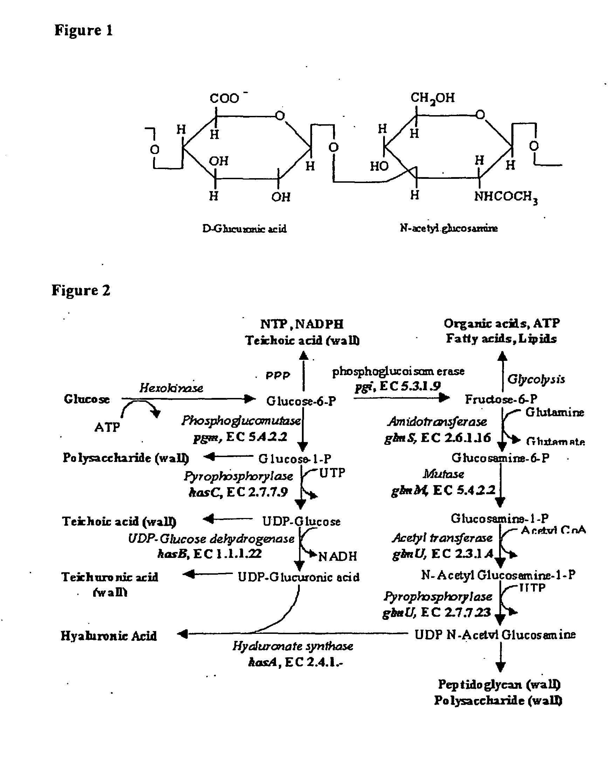

The term "hyaluronate" refers to the conjugate base of hyaluronic acid (HA). Because the molecule typically exists *in vivo* in its polyanionic form, it is most commonly referred to as "hyaluronan." HA is comprised of linear, unbranching, polyanionic disaccharide units consisting of glucuronic acid (GlcUA) an N-acetyl glucosamine (GlcNAc) joined alternately by beta 1-3 and beta 1-4 glycosidic bonds, as shown in Figure 1. HA is a member of the glycosaminoglycan family, which includes chondroitin sulphate, dermatin sulphate and heparan sulphate. Unlike other members of this family, it does not covalently bind to proteins.

In a neutral aqueous solution, due to the hydrogen bond formation, water molecules and adjacent carboxyl and N-acetyl groups impart a conformational stiffness to the polymer, which limits its flexibility. The hydrogen bond formation results in the unique water-binding and retention capacity of the polymer. It also follows that the water-binding capacity is directly related to the molecular weight of the molecule. It is known that up to six liters of water may be bound per gram of HA. (Sutherland, Novel and established applications of microbial polysaccharides. Trends Biotechnol 16:41-46, 1998).

Hyaluronic acid (HA) and its derivatives have been extensively studied for their applications in biomedical practices. The biocompatibility characteristic of this polymer has attracted attention of orthopedics as it can be used in viscosurgery and allows surgeons to safely create space between tissues. Viscosurgical implants are constructed from HA. (Balazs, Aug 1993, *id*.). HA's viscoelastic character has been used to supplement the lubrication in arthritic joints. It has also been used for targeted drug delivery as a microcapsule. Finally, because of its high water retention capacity, this EPS (extracellular polysaccharide) also occupies a niche in the lucrative cosmetics market. In 2003 the FDA approved hyaluronan injections for filling soft tissue defects such as facial wrinkles. Restylane® is a common trade name for the product. Hyaluronan injections temporarily smooth wrinkles by adding volume under the skin, with effects typically lasting for six months. In addition, HA solutions are characteristically viscoelastic and pseudoplastic. These characteristics are found even in very dilute solutions of this polymer where very viscous gels are formed. The viscoelastic property of HA solutions, which is important to its use as a biomaterial, is controlled by the concentration and molecular weight of the HA. The molecular weight of HA from different sources is highly variable ranging from 10⁴ to 10⁷ Da. The extrusion of HA through the cell membrane as it is produced permits unconstrained polymer elongation, which can result in a very high molecular weight of HA.

Molecular weight is a parameter that most affects the physical properties of hyaluronic acid-based medical devices. Molecular weight influences the viscosity versus concentration profiles of hyaluronic acid solutions, and plays a major role in its other visco-elastic properties. In particular, solutions that require a given viscosity for therapeutic efficacy require lower concentrations of hyaluronic acid if the molecular weight is increased. This has been shown to provide advantages in both ocular and orthopedic applications. A process that is capable of generating medical grade hyaluronic acid having a molecular weight greater than 750,000 daltons thus has significant commercial potential. In many pharmaceutical applications, it is undesirable to have low molecular weight hyaluronic acid in the formulation, for example in view of the inflammatory effects of low molecular weight HA as reported in U.S. Pat. No. 4,141,973

Hyaluronic acid is used in some top selling commercial products, including Synvisc (Genzyme), Orthovisc (Anika), Seprafilm (enzyme), Restylane (Q-Med), Healon (Pharmacia), Amvisc (Med-Chem) *etc.* Due to its high cost, however, it is only used in very small concentrations in these products. HA has more commercial value than other microbial EPS. With an estimated world market value of $US 500 million, it is sold for about $US 100 000 per kilogram.

HA has been conventionally extracted from rooster combs and bovine vitreous humor. However, it is difficult to isolate high molecular weight HA at industrially feasible rate from these sources, because it forms complex with proteoglycans present in animal tissue (O'Regan et al., 1994). It is presently impractical to control the molecular weight of the biopolymer while it is synthesized in animal tissue. Moreover, the use of animal-derived biochemicals for human therapeutics has raised ethical issues, and is met with growing resistance. Besides ethical issues, there is a potential infectious disease risk associated with the use of animal-derived biochemicals for human therapeutics. Given that 10 MDa chains could be obtained from animal tissue, there is considerable scope for improvement.

The above discussed drawbacks have forced the industry to adopt to bacterial fermentation processes with the hope of obtaining commercially viable biopolymer. Using a bacterial fermentation process, HA is released into the growth medium, which helps in controlling the polymer characteristics and results in improved HA yields. The amount of biopolymer that can be produced by the above route is theoretically unlimited. The use of bacterial fermentation does have, however, disadvantages. The recent trend is to use Lancefield's group A and C *Streptococci,* which naturally produce a mucoid capsule of HA. The HA capsule is a biocompatibility factor that enables this gram positive bacteria to evade host immune defenses and accounts for its characteristically high virulence level.

Previous subsequent improvements in the extraction and purification processes using bacteria have resulted in an inherent molecular weight reduction of HA. *Streptococcal* fermentations have been only able to produce HA with an average molecular weight in the range of 1 to 4 MDa. As discussed above, high molecular weight is a desirable property of the HA biopolymer. The present disclosure provides methods for obtaining HA having a high molecular weight.

*Streptococci* are nutritionally fastidious, facultative anaerobes, which produce lactic acid as a by-product of glucose catabolism. Hence the energy recovered by these bacteria is lower relative to aerobic bacteria. The yield of HA from bacterial fermentation obtained using previously known methods is characteristically low (0.1 g/g glucose, 0.15 g/lh). Such low yields certainly struggle to meet market demand. In addition, strict nutritional requirements for fermentation influence economics by prohibiting the use of chemically defined media for production scale fermentations and limit the choice of complex media that can be employed.

Several groups have provided proposed process parameter optimization, selection of process mode of HA, yield optimization by complex medium design, and model based fermentation control of HA. Cooney, M.J., Goh, L.T., Lee, P.L., and Johns, R.R., Structure model-based analysis and control of the hyaluronic acid fermentation by Streptococcus zooepidemicus: Physiological implications of glucose and complex-nitrogen-limited growth, Biotechnology Progress, 15: 898-910 (1999); Lars M. Blank, Richard L. McLaughlin, Lars K. Nielsen, Stable production of hyaluronic acid in Streptococcus zooepidemicus chemostats operated at high dilution rate, Biotechnology and Bioengineering, 90(6): 685 - 693 (2005); Johns, M. R., Goh, L.-T., and Oeggerli, A., Effect of pH, agitation and aeration on hyaluronic acid production by Streptococcus zooepidemicus. Biotechnology Letters 16: 507-512 (1994); Kitchen, J. R., and Cysyk, R. L., Synthesis and release of hyaluronic acid by Swiss 3T3 fibroblasts, Biochemical Journal, 309: 649-656 (1995); Armstrong D, Johns MR, Culture conditions affect the molecular weight properties of hyaluronic acid produced by Streptococcus zooepidemicus, Appl. Environ. Microbiol., 63: 2759-2764 (1997).

Over time, HA molecular weight has become major area of study for researchers. Researchers, such as Armstrong and Johns, have examined the effect of process parameters to ascertain effects on the molecular weight of HA. Armstrong, D. C., and Johns, M. R., Effect of Culture Conditions on Molecular Weight of Hyaluronic Acid Produced by Streptococcus zooepidemicus, Applied and Environmental Microbiology, 63: 2759-2764 (1997); Armstrong, D. C., and Johns, M. R., Improved molecular weight analysis of streptococcal hyaluronic acid by size exclusion chromatography, Biotechnology Techniques, 9: 491-496 (1995).

These groups have found that certain fermentation parameters affect the molecular weight of produced HA. Armstrong et al (1997) reported that conditions such as temperature, aeration and glucose concentration affect the molecular weight of HA whereas pH and agitation do not. It was found that low growth temperatures (28 C), culture aeration and high initial glucose concentration (40 g/l) resulted in the production of higher molecular weight HA in *S. zooepidemicus.* Previous groups also found that culture pH and agitation did not affect the molecular weight outcome of HA fermentations. Specifically, Johns et al. (1994) previously reported that pH and agitation affected the production of HA generally, but not molecular weight.

It is already known fact that specific growth rate of the bacteria and the molecular weight of HA are inversely proportional. This effect may be explained by a resource-based metabolic model for HA synthesis, as illustrated in Figure 2. In its most simplistic form, two competing processes occur within a bacterial cell, i.e., cell growth and biosynthesis of HA. These two processes compete for the limited resources, such as carbon, nitrogen and energy. At low specific growth rates, the cell directs more glucose-derived activated precursors (namely UDP-Glc and UDP-GlcNAc) to HA synthesis rather than cell wall synthesis. The higher ATP yield from aerobic glucose catabolism favors the formation of UTP, which is required for the formation of the two activated precursors of HA synthesis, namely UDP-GlcUA and UDP-GlcNAc. Glucose, which may be used to synthesize HA, is also depleted by lactate production under anaerobic growth.

The specific rate of HA production (g g⁻¹ h⁻¹) was also previously observed to increase with decreasing specific growth rate. Given the fact that the density of active HA-synthesizing enzymes is unlikely to change, the higher rate of production can be attributed to a higher polymerization rate through each synthase. An increased synthase activity may arise from the higher intracellular substrate concentration resulting from low growth rate.

The molecular weight of the HA produced is determined by the number of precursors that the synthase is able to polymerize during its lifetime. Based upon this theory, overexpression of the synthase results in decrease in the molecular weight of HA synthesized. Indeed it may reduce the mean molecular weight because there are more enzymes competing for the same resources. A similar effect was reported for the overexpression of polyhydroxybutyrate synthase in recombinant *E. coli.* (Sim et al., PHA synthase activity controls the molecular weight and polydispersity of polyhydroxybutyrate in vivo," Nature Biotech. 15: 63-67, 1997).

U.S. Pat. No. 4,897,349 demonstrated that improved overall yields of HA could be obtained by controlling the oxygen available for metabolism by a hyaluronic acid producing microorganism. The available oxygen was limited to stimulate a disproportionately larger production of the desired hyaluronic acid. The culture conditions with respect to carbon source was not studied. However, the process required modification of dissolved oxygen, which did not usually result in consistent yields of HA produced.

European patent EP 0694616 employed a concentration of 2.5% of a culture of *Streptococcus zooepidemicus* obtained from a collection. The fermentation took about 24 hours at 37 °C, with the pH maintained at 7.00 by continuous automatic addition of NaOH. 33% of the dextrose was added at the moment of inoculation, and the remainder was administered continuously. The yield of the process, as determined by HPLC analysis, was as low as 0.8 g/l with a mean molecular weight estimated at around 500,000 Da.

Japanese patent JP62215397 demonstrated that a microorganism capable of producing hyaluronic acid was aerobically cultured in a medium under agitation, as saccharides were added to the medium several times, the concentration of the saccharides was always kept at 0.1-3 w/v% in the medium. Hyaluronic acid accumulated in the medium was collected. The preferred saccharide was glucose or lactose. The hyaluronic acid accumulated in the culture mixture was separated by a conventional method for separation of polysaccharides. The process presented in this patent was a fed batch process and not applicable to a continuous batch process.

Japanese patent JP62257901 disclosed a process for producing HA that could be obtained by culturing hyaluronic acid-producing bacteria in a carbohydrate-containing medium (e.g., with a pH 7.5 containing 0.50% of yeast extract, 0.75% of peptone, 0.02% of sodium sulfite, and 1-3% of glucose).

Japanese patent JP62289198 presented a process wherein a hyaluronic acid-producing microbial strain such as Streptococcus equi NK-850214 was inoculated in a liquid medium containing arginine and/or glutamic acid at a concentration of 0.01-1 wt%, in addition to peptone, NaCl, trace vitamins, etc., by a culture process comprising the addition of ≥ 20g carbohydrate, such as glucose per 1 liter of the medium, in ≥ 3 divided doses in a manner that constantly kept the carbohydrate concentration at 0.1-3 w/v% throughout the culture.

In Japanese patent JP05276972, a microorganism capable of producing hyaluronic acid was cultured in a culture solution containing a surfactant. Hyaluronic acid was formed and accumulated in the culture solution and collected.

Japanese patent JP06038783 provided a process of producing hyaluronic acid on an industrial scale by culturing a hyaluronic acid-producing microbial strain belonging to the genus *Streptococcus* in a nutrient medium. The medium contained a specific amount of a sugar component (as the main carbon source) under specific conditions while stirring the medium by aeration. A microbial strain belonging to the genus *Streptococcus* and capable of producing hyaluronic acid (e.g., *Streptococcus zooepidemicus*) was inoculated on a nutrient medium containing ≥ 3% sugar component as a main carbon source and was cultured at pH 7 and 33°C for 4 days under stirring by aeration. The yield obtained by this process was 2.1 g/L.

Japanese patent JP06319579 presented the production of hyaluronic acid in a medium containing glucose and fructose as major carbon source to accumulate hyaluronic acid of high molecular weight in the culture mixture.

U.S. Pat. No. 4,784,990 demonstrated a method for obtaining sodium hyaluronate comprising growing with vigorous agitation a microorganism of the genus *Streptococcus* under appropriate conditions in a nutrient medium containing a sugar component as a carbon source. The microorganism produced large amounts of high molecular weight hyaluronic acidand this patent concerns a method for selecting microorganisms which produce enhanced amounts of hyaluronic acid and which lack hemolytic activity. S. zooepidemicus HA-116 ATCC 39920 which is a mutant strain. The sodium hyaluronate was then recovered by removing the microorganism and other materials insoluble in the medium, precipitating the sodium hyaluronate from the medium, e.g. using organic solvents, and recovering the precipitate. The precipitate was then ground and dried. Compositions of sodium hyaluronate characterized by an absence of pyrogenicity and inflammatory activity could be produced by these methods. The medium had a substantially constant pH between about 6.0 and 7.5 and the sodium hyaluronate excreted into the medium by the organism was purified using methods involving precipitation, redissolving and reprecipitating the hyaluronate. The sodium hyaluronate could be precipitated from the medium or filtrate by adding a first organic solvent, such as isopropanol, to the medium. The precipitate was redissolved in 3% aqueous sodium acetate and then reprecipitated with a second organic solvent such as ethanol. The second precipitate was redissolved in 3% aqueous sodium acetate and activated charcoal was added to form a suspension. The suspension was filtered and a third organic solvent, e.g. acetone, was added to produce a precipitate of sodium hyaluronate. The first, second and third organic solvents could each be isopropanol, ethanol or acetone. Alternatively the hyaluronate could be precipitated by the same organic solvent in each step. For example, sodium hyaluronate was precipitated from the medium by using isopropanol in all three of the precipitation steps. The process in this patent for medical grade HA involves use of detergents such as cetyl pyridinium chloride, multiple solvent precipitation steps, florisil treatment etc.

U.S. Pat. No. 4,946,780 provided a method of producing HA from fermentation by employing surfactants such as cetyl pyridinium chloride. The method also involved a purification procedure comprising contacting the solution containing sodium hyaluronate with alumina, activated charcoal or mixtures thereof and then with silica to form a purified solution of sodium hyaluronate. The method then added alcohol to the purified solution of sodium hyaluronate to precipitate purified sodium hyaluronate and dried the purified sodium hyaluronate. Surfactants are difficult to get rid of from the product and necessitate multiple washing steps with pyrogen-free water

U.S. Pat. No.. 5,563,051 disclosed a process wherein after the fermentation process, the biomass was killed with a particularly suitable agent. The agent used to kill the biomass, was formaldehyde, e.g., in the form of the aqueous solution commonly known as formalin. The HA was extracted with an aqueous medium containing an anionic surfactant ,i.e., sodium dodecyl sulphate. The patent further disclosed the use of an ultra filtration membrane with an appropriate molecular weight cut off which was usually from 10,000 to 25,000 Daltons, and preferably 20,000 Daltons. The filtered solution containing the dissolved HA was diafiltered with 8 to 20 volumes of purified water, preferably about 10 volumes of purified water, where the filtrate was continuously discarded.

U.S. Pat. No. 6,489,467 claimed a process for purifying high molecular weight hyaluronic acid from a biological source, including the steps of adjusting the pH of an aqueous solution containing high molecular weight hyaluronic acid from a biological source to a pH in the range from 1.7 to 3.3. The process further involved diafiltering the aqueous solution at the same pH using a filter having a pore size in the range from 100,000 Daltons nominal molecular cut-off to 0.45 □, and removing cells from the aqueous solution containing high molecular weight hyaluronic acid from biological source.

U.S. Pat. No. 7,002,007 disclosed methods involving contacting a hyaluronate-containing source with an acid to make an acidic hyaluronate suspension, contacting that suspension with an anionic exchange medium in the presence of an acidic buffer, and thereafter contacting the medium with an acidic buffer having a higher salt content to desorb the hyaluronate from the medium.

Previous methods for producing HA usually use glucose as a carbon source. Using these methods, the molecular weight of the HA obtained often was found to be between 2 and 4 x 10⁵ Da, i.e., a relatively low molecular weight. Moreover, the inherent viscosity of HA increases with the increase in its molecular weight. This increased viscocity has presented a major obstacle during fermentation, as well as in purification of HA. Although high molecular weight HA with may be too large to penetrate the skin and the bloodstream, it has a number of valuable uses. In in topical preparations, for example, high molecular weight HA is useful in eye surgery and in cosmetics. Likewise, high molecular weight HA can be used in a viscoelastic film to retain skin moisture and to block foreign substances.

WO 86/04355, WO 92/08799 and WO 00/44925 describe processes of producing or purifying HA.

Therefore, it has become a challenge and a necessity to provide a process for producing high molecular weight HA, including that which meets specifications set for medical applications, e.g., as described in British Pharmaacoepia, 2003.

Previous methods for purifying HA have involved at least three successive solvent precipitation steps, thereby resulting in an increased number of process steps and an overload on the lyophizer. Previous methods for precipitating fermentation broth also involved the use of surfactants or detergents, such as cetyl pyridinium chloride/hexadecyltrimethyl ammonium bromide. The removal of residual detergent or surfactant increases number of processing steps needed, it has posed complexity and cost.

In addition, previously known purification procedures, such as diafiltration steps employed to achieve the high molecular weight HA involve the use of large volumes of organic solvent. The use of such solvent creates handling problems in scale up operations and results in the environmental hazards.

The present invention relates to improvements on methods for purifying HA, such as high molecular weight HA and its salt, including that having medical applications as in British Pharmaacoepia, 2003. The present invention presents a purification process of hyaluronic acid that includes silica gel filtration combined with active carbon treatment and subsequent diafiltration using less amount of solvent, thereby yielding a better quality of hyaluronic acid having biomedical applications. Likewise, the present invention provides a commercially viable purification process of hyaluronic acid and its salts thereof, having industrial applications.

### OBJECT OF THE INVENTION

It is the object of the present invention to provide purification process of hyaluronic acid, yielding a better quality of hyaluronic acid having biomedical applications.

It is the object of the present invention to provide an efficient process for removal of protein impurities from hyaluronic acid by employing two steps of silica gel purification and carbon treatment.

It is the object of the present invention to provide an efficient process for diafiltration using less amount of solvent in the purification of hyaluronic acid.

It is the object of the present invention to provide a high molecular weight hyaluronic acid of medical grade.

It is the object of the present invention to provide a process that is free from surfactants or detergents.

It is the object of the present invention to provide a single cycle process for purification of hyaluronic acid.

It is the object of the present invention to provide a process, which comprises minimal amount of solvents.

It is the object of the present invention to remove at least 90% of the protein impurities employing silica gel and activated carbon treatment.

It is the object of the present invention to provide a process for constant volume diafiltration resulting in high molecular weight HA.

It is also the object of the present invention to provide HA as per specifications of British Pharmacoepia 2003

### SUMMARY OF THE INVENTION

The present invention yields a better quality of hyaluronic acid having biomedical applications, free from surfactants or detergents.

According to the present invention the following steps for the production of medical grade hyaluronic acid can be used.
a) Preparation of HA by fermentation
b) Removal of protein impurities
c) Diafiltration

HA can be prepared by initial culturing of the microorganism *Streptococcus zooepidemicus,* in a suitable medium followed by fermentation of the broth in a I-10 Liter fermentor at about 30-40 °C, with agitation at about 300-500 rpm and 1-3 vvm (vol/vol/min) aeration for about 15-28 hours by maintaining the pH at a neutral range. (Aeration rate vvm = gas volume flow per unit of liquid volume per minute (volume per volume per minute)). After incubation, the fermentation broth is suitably diluted with water and clarified. The HA present in the clarified broth is reprecipitated with equal volumes of suitable solvent including but not limited to isopropanol. The precipitated high molecular weight HA is then converted to its salt wherein it can be then solubilised for subsequent purification procedure. The HA can be converted into its sodium salt by the addition of 3% sodium acetate and homogenized until complete dissolution.

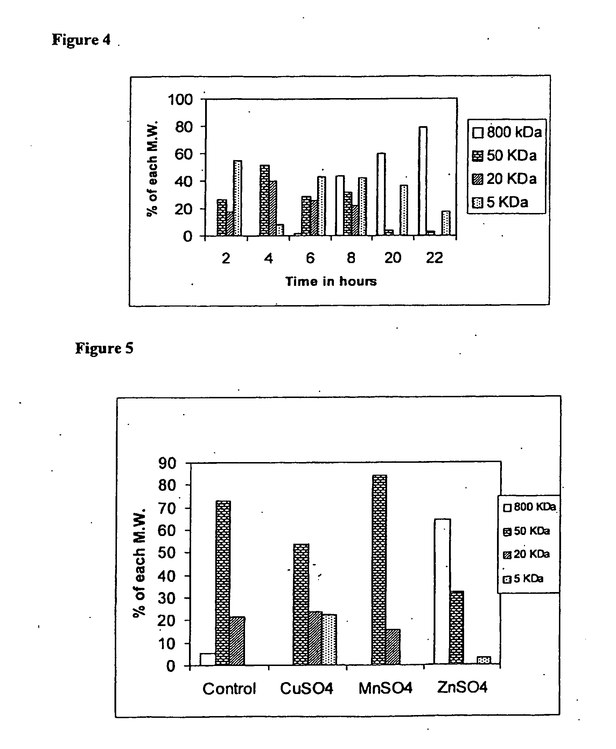

The culture conditions described herein optimize the yield and molecular weight of HA. It has been found that production of HA is a growth associated phenomenon. One can produce HA with high molecular weight by controlling culture conditions. For example, the molecular weight of produced HA increases with bacteria growth. At the beginning of fermentation process, lower molecular weight population (∼ 5000 Da) is about 60 % of total HA. About 70 % of total HA is of high molecular weight (> 800 KDa) by the end of 22 h of fermentation (Figure 4).

In addition, one may use different metals in the fermentation process of HA. Metal ions are known to enhance capsule production in various bacteria. The effect of CuSO₄, MnSO₄ and ZnSO₄ at 0.025 % (final conc.) was tested on the yield of HA at the end of 24 h of fermentation. While there was no significant difference between the control (no metal addition) and the flasks treated with CuSO₄ or MnSO₄, a significant portion (60%) of total HA in the medium treated with ZnSO₄ was found to be in the higher molecular weight region (>800 KDa) (Figure 5).

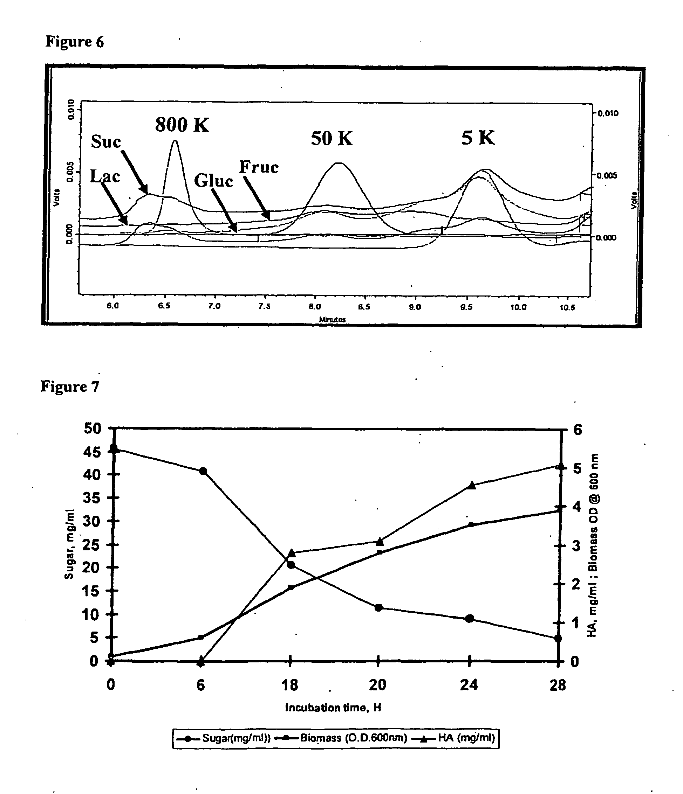

Likewise, the carbon source, i.e., sugar, in the culture medium may be varied. The growth and HA production in medium containing various types of sugars were tested. Different sugars in culture medium affected the molecular weight of HA during production. While growth was fairly constant in most of the sugars (2 % final conc.), lactose and sucrose gave better yield of high molecular weight HA (>800 K) as compared to glucose, which gave HA of lower molecular weight (Figure 6). The yield of HA obtained using sucrose was at least 10-fold higher and molecular weight of HA was found to be much higher, as compared to glucose. As shown in Figure 6, the molecular weight of HA using sucrose as described herein was greater than 8 x 10⁵ Da (i.e., 800 kDa), while in glucose medium, the molecular weight was found to be between 2 and 4 x 10⁵ Da (i.e., 200-400 kDa).

The effect of media on the production of high molecular HA was examined. In one experiment, sugar and casein hydrolyzate enzyme content in the medium were varied. In thisexperiment, sugar provided necessary energy and casein enzyme hydrolysate provided a necessary nitrogen source. Figure 7 shows results when the sugar concentration was increased from 2 % to 5 % and when casein hydrolyzate enzyme concentration was brought down to 1 % from 2.5 % in the medium.. The parameters provided an increased yield of 5-6 g/L of HA (Figure 7). "Sugar" in Figure 7 refers to sucrose; "biomass" refers to the cell density, which reflects growth of the microorganism. The yield was less when both the sugar concentration was lower (2%) and casein enzyme hydrolysate concentration was higher (2.5%), or when only the sugar concentration was increased to 5% (data not shown).

HA can be prepared by initial culturing of the microorganism *Streptococcus zooepidemicus,* in a suitable medium and subsequent fermentation of the broth in a 1-10 Litre fermentor at about 30-40°C, with agitation at about 300-500 rpm and 1-3 vvm aeration for about 15-28 hours by maintaining the pH at a neutral range.

In one embodiment, the present invention provides an efficient process for removal of protein impurities from hyaluronic acid. The present invention employs silica gel filtration and active carbon treatment for efficient removal of protein impurities..

In one embodiment the present invention provides an efficient process for purification of Hyaluronic acid by diafiltration. The present invention employs less amount of solvent in the process.

After incubation, the fermentation broth is suitably diluted, typically with water, and clarified. The HA present in the clarified broth is typically reprecipitated with equal volumes of suitable solvent including but not limited to isopropanol.

The precipitated high molecular weight HA is then typically converted to its salt wherein it can be then solubilized for subsequent purification procedure. The present invention typically converts the HA into its sodium salt by the addition of 3% sodium acetate and homogenized until complete dissolution.

The present invention also provides an efficient purification process that removes protein impurities from hyaluronic acid. The process employs silica gel filtration and active carbon treatment for efficient removal of protein impurities. The initial treatment with silica gel typically removes about 68-70% protein. Typically, after removal of silica gel by centrifugation, the high molecular weight HA is treated with active carbon, which then removes 85-90% of the protein. In one embodiment, the process involves passing the silica treated sample through a carbon impregnated on cellulose cartridge.

In one embodiment, the solution obtained after removal of protein impurities is further purified by diafiltration. The present invention employs less amount of solvent in the process. For example, in one embodiment, the diafiltration step involves dilution of the HA solution with the solvent, i.e. pyrogen-free water, and the dilution is done only 5 times, as compared to 10 times, as described in U.S. Pat. No. 6,489,467 (Carlino et al. (2002), Process for purifying high molecular' weight hyaluronic acid) thereby significantly reducing the volume handling. A continuous mode of diafiltration may be used, which involves dilution with sterile, pyrogen free water, for example five times. In one embodiment, the process further involves isolate sterile, purified hyaluronic acid by filtering through a 0.22 µm pore filter. In another embodiment, sodium hyaluronate product for biomedical purposes can be obtained by aseptic filtration.

Optionally, the sodium hyaluronate can be reprecipitated with isopropanol to yield the purified high molecular weight HA. The HA obtained can be lyophilized until the moisture content is less than 5 %.

In sum, the present invention provides an improved purification process for HA that does not employ acidic pH conditions, and does not use any detergents nor surfactants. The removal of protein impurities step does not employ any hazardous chemical, such as formalin. In addition, the purification process employs less solvent dilutions and therefore reduces the load on the lyophilizer.

### BRIEF DESCRIPTION OF THE DRAWINGS

The following drawings form part of the present specification and are included to further demonstrate certain aspects of the present disclosure. Thus, the invention can be better understood by reference to one or more of these drawings in combination with the detailed description of embodiments presented herein.
Figure 1: Shows disaccharide repeating unit of HA comprising GlcUA and GlcNAc.
Figure 2: Shows the HA biosynthetic pathway in *Streptococci.*

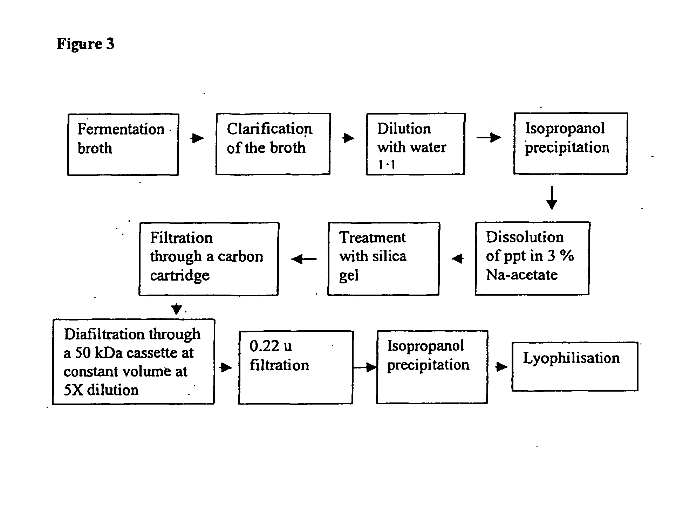

Figure 3: Illustrates the flow sheet of the process for preparation of high molecular weight hyaluronic acid of medical grade.
Figure 4. Distribution of HA of various molecular weight during the growth of S.
*zooepidemicus.*
Figure 5. Effect of metal ions on population of HA of varying molecular weights.
Figure 6. HPLC profile of HA from media containing different carbon sources. Culture medium was supplemented with glucose, sucrose, lactose or fructose at a final concentration of 2 %.
Figure 7. Medium optimization for increased yield of HA. Initial sucrose content was 5%, and the concentration of casein hydrolyzate was 1.0%. The "%" corresponds to g/100 ml of medium; "sugar" refers to sucrose; and "biomass" is the cell density reflecting growth of the microorganism. Casein hydrolyzate enzyme acts as a nitrogen source during growth of the bacteria.

### DETAILED DESCRIPTION OF THE INVENTION

The HA production methods described herein provide high yield of medical grade high molecular weight HA from bacterial fermentation broth. The present invention provides a cost effective methods for purifying HA.

Definitions:
The term " hyaluronic acid" or "HA" as used herein indicates hyaluronic acid obtained from any biological source.
The term "hyaluronate" as used herein refers to any salt form of HA, such as the sodium salt of HA.
The term "high molecular weight" as used herein refers to HA or a salt of HA having a molecular weight of at least 1000 KDa. (1 million Da)
The term "silica gel" means a porous, granular form of silica, synthetically manufactured from sodium silicate with varying porosities and remains the highest capacity adsorbent available. One example is a silica gel sold by Ineos.
The term "active carbon" means a material with an exceptionally high surface area. Just one gram of activated carbon has a surface area of approximately 500 m², typically determined by nitrogen gas adsorption, and includes a large amount of microporosity. One example is sold by Millipore (Millistak +, Activated Carbon minicapsule M40AC23HH3).
The term "diafiltrating" or "difiltration" refers to a crossflow filtration process allowing for the transfer of low molecular weight species, water and/or solvents through a membrane without changing the solution volume. This process is used for purifying retained large molecular weight species, increasing the recovery of low molecular weight species, buffer exchange and simply changing the properties of a given solution. It can be performed, for example, by Sartocon slice cassette mod. No. 3051465001E-SG, 50 kDa MWCO.
The term "neutral pH" means pH is 7.0
   The microbial source is typically a *Streptococcus* species capable of producing high molecular weight hyaluronic acid. For example, the microbial source may be selected from *Streptococcus zooepidemicus, Streptococcus equi* and *Streptococcus pyogenes.*
The term "solvent" used herein indicates any solvent organic or inorganic solvents. In one embodiment, the solvent is isopropanol.

As noted previously, production of HA is a growth associated phenomenon and is inversely related to the growth. Typically, culturing and fermentation parameters are selected that balance these two processes.

The molecular weight of HA increases over time in culture, with smaller chains being detected early in the growth phase followed by high molecular weight HA being detected during the latter part of growth. Previous studies, such as those by Armstrong and Johns, have suggested that temperature, pH and agitation speed affect molecular weight of HA drastically when producing HA in bacteria. When comparing results with that of the ' study carried out by Armstrong *et al.* (Armstrong and Johns, 1997) and other conventional processes, other researchers found that the culture pH and agitation speed did not affect the molecular weight of HA drastically during production

In the HA production process described herein, the use of lower temperature and aeration, amongst other parameters, can improve the yield of HA production, and increase the molecular weight of HA that is produced.

Previous studies found that initial glucose concentration had a profound effect on the molecular weight of HA. One group, Armstrong and Johns, offered an explanation, i.e., that when the activated sugar monomers UDP-GlcUA and UDP-GlcNAc were present at high concentrations, HA chain elongation persisted. The external glucose concentration might have been linked to the internal concentrations of these monomers, because they were derived from glucose and their synthesis requires substantial energy consumption. (Armstrong and Johns, 1997).

The present inventors have found that sucrose and lactose are better carbon sources than glucose for production of high molecular weight HA. Because sucrose and lactose are disaccharides, they may serve as better energy source than glucose. Typically, in the HA production process described herein, growth on sucrose or lactose at a higher concentration, and protein, such as casein hydrolyzate, at a lower concentration, generates 5-6 g/L of high molecular weight HA. The molecular weight of HA was found to be in the range of 3.5 - 4.0 x 10⁶ Da.

The effect of various metal ions on the molecular weight of HA produced have also been studied. The metal ions present in the culture medium are responsible for oxidative stress to the bacteria whereby free radicals are being released during the process, which can have a bactericidal effect. In order to circumvent, several bacteria, particularly some *Pseudomonas* species produce EPSs (extracellular polysaccharides) such as alginates, etc. which helps the bacteria top invade the host and thus survives. Keith LMW, Bender CL. AlgT (ζ22) Controls Alginate Production and Tolerance to Environmental Stress in Pseudomonas syringae, J. Bacteriol., 181: 7176-7184 (1999).

At least one group has suggested that streptococcal and mammalian hyaluronate synthases prefer magnesium ion to other metal ions for the production of high molecular weight of HA. (Yamada and Kawasaki, Microbial synthesis of hyaluronan and chitin: new approaches. J. Biosci. Bioeng. 99: 521-528, 2005). Yamada T and Kawasaki, 2005 provided another novel HA producing Chlorella-virus system stimulated by manganese ions, which produced about 0.5-1 g/L of HA. In the HA production process described herein, metal ions such as Cu and Mn do not induce the production of high molecular weight HA, although significant population of HA in the medium containing Zn were in the high-molecular weight range.

To summarize, the HA production process described herein, has optimized the culture conditions that have resulted in improved yield , such as 5-6 g HA/L, of high molecular weight HA. Typically, casein hydrolyzate enzyme is lowered and the sugar concentration is increased in the medium. These conditions result in lower growth rate. Because production of HA is inversely proportional to growth, increase in HA yield is obtained. Typically, the molecular weight of HA obtained by the present invention is about 3.5 x 10⁶ to 3.9 x 10⁶ Da after 24 h of fermentation.

A process for producing high molecular weight hyaluronic acid (HA) or its salt having a molecular weight of at least 1 million Da, comprises: (a) providing a bacteria capable of producing HA; (b) providing a nutrient medium comprising a divalent metal ion salt (such as MgSO₄, CuSO₄, MnSO₄, and ZnSO₄), casein hydrolyzate, and a carbon source (such as lactose or sucrose); (c) culturing the bacteria in the nutrient medium; and (d) fermenting, such as continuous fermenting, the nutrient medium under aerobic conditions. Bacteria capable of producing HA include those selected from the group consisting of *Streptococcus zooepidemicus, Streptococcus equi and Streptococcus pyogenes.*

Typically, the nutrient medium comprises 1% casein hydrolyzate and/or 5% of lactose or sucrose. Typically, the nutrient medium comprises zinc, such as in the form of ZnSO₄, casein hydrolyzate at a concentration of 10 g/L, and a carbon source selected from the group consisting of lactose and sucrose at a concentration of 50 g/L. Typically, the fermenting step is conducted for at least 15 hours at a temperature of 30-40 °C, an agitation speed of 300-500 rpm, an aeration of 1-3 vvm, and at neutral pH.

Typically, the process produces HA or its salt having a molecular weight in the range of about 3.5×10⁶ Da to 4.0×10⁶ Da. Typically, the process yields at least 5 g of high molecular weight HA per liter of nutrient medium. Typically, the molecular weight of the HA is at least 3 x 10⁶ Da after 24 hours of fermenting.

The present invention provides a method for the removal of protein impurities from produced HA, which comprise the two steps of silica gel treatment followed by active carbon treatment. A subsequent diafiltration step also helps ensure a highly purified salt of HA. The product thus obtained can be then aseptically filtered for biomedical applications.

Previously known purifying processes precipitate HA using detergents or surfactants, such as cetyl pyridinium chloride/hexadecyltrimethyl ammonium bromide. This requires, however, multiple steps to remove the residual detergent or surfactant. The present invention provides an efficient purification method for HA, which does not employ detergents or surfactants.

Previously known processes for purifying HA using diafiltration techniques have involved a high number of dilutions with solvent, such as diluting 10 times. *See* U.S. Pat. No. 6,489,467 (Carlino, et al., Process for purifying high molecular weight hyaluronic acid (2002)). The use of high volumes of solvent creates problems during handling at a larger scale, such as bigger processing vessels and longer time to complete the process and of course higher cost of production. The present invention avoids such problems by using fewer dilutions.

The present invention provides a process for purifying hyaluronic acid (HA) or its salt from a bacterial fermentation broth comprising (a) diluting and clarifying the fermentation broth;(b) precipitating the HA present in the broth with a equal volume of solvent, such as isopropanol, ethanol or acetone, preferably isopropanol; (c) dissolving the precipitated HA or its salt in a solution, such as 3% sodium acetate; (d) adding silica gel to the HA solution or HA salt solution from step (c) and then removing the silica gel, such as by centrifugation; (e) treating the HA solution or HA salt solution from step (d) with active carbon; and (f) diafiltrating the HA solution or HA salt solution from step (e) using about 5 volumes of solvent, such as sterile pyrogen-free water ; wherein the process is conducted in the absence of any detergent or surfactant or formalin, and at neutral pH.

In one embodiment, the process further comprises converting the HA precipitated in step (b) to its salt, and then homogenizing the HA salt in the solution of step (c). In another embodiment, the active carbon is impregnated on cellulose cartridge in step (e). In another embodiment, the process further comprises isolating sterile, purified HA or its salt by filtering through an aseptic filter; and/or lyophilizing the HA or its salt until the moisture content is less than 5%.

The following examples are included to demonstrate preferred embodiments of the invention. It should be appreciated by those of skill in the art that the techniques disclosed in the examples which follow represent techniques discovered by the inventor to function well in the context of the invention, and thus can be considered preferred. However, those of skill in the art should, in light of the present disclosure, appreciate that many changes can be made in the specific embodiments which are disclosed and still obtain a like or similar result. Examples describing the production of HA are included for reference purposes.

### EXAMPLE 1: Bacterial strain and media. Streptococcus equi subsp. zooepidemicus ATCC 39920 was obtained from American Type Culture Collection. The bacteria were maintained on brain heart infusion agar or tryptic soy broth.

### EXAMPLE 2: Estimation of HA

HA was routinely estimated by the carbazole assay (Bitter, T., and Muir, M., A modified ironic acid carbazole reaction. Anal Biochem 4: 330-334, 1962) in the fermented broth after precipitation with equal volume of isopropanol and redissolving in 3 % Na-acetate solution.

### EXAMPLE 3: Media optimization

For media optimization experiments, the S. *zooepidemicus* was grown in a media constituting 2.5% casein hydrolyzate enzyme, 1% yeast extract, 0.2% K₂HPO₄, 0.15% NaCl, 0.04% MgSO₄.7H₂O, and 2% carbon source (sucrose). The organism was grown in Erlenmeyer flasks at 37°C at 200 rpm for 24 h. For evaluating the effect of metal ions on HA production, filter sterilized solutions of CuSO₄, ZnSO₄ and MnSO₄ were added to the medium at final concentration of 0.025%. *See* Figure 5.

### EXAMPLE 4: Molecular weight determination

The molecular weight of HA was determined by size-exclusion chromatography on HPLC using a Shodex OH-Pak SB805-804HQ columns connected in series. The mobile phase used was 1 M NaNO₃ at 1ml/min flow rate and peaks were detected using a RI detector. The column was calibrated with pullulan standards of varying molecular weights.

### EXAMPLE 5: Removal of protein impurities

### Silica gel treatment

A portion of the homogenized suspension, 100 ml, was treated in batch mode, with silica gel at 2 % final concentration for adsorption of protein. This step removes 68-70 % protein. The silica gel is separated from the sodium hyaluronate solution by centrifugation at 12,000 rpm for 20 min at 4°C.

### Active carbon treatment Further treatment of high molecular weight hyaluronic acid solution was done with charcoal adsorbed 0.45µ filter assembly (Millipore, Millistak +, Activated Carbon minicapsule M40AC23HH3). The flow rate was 14ml / min. This step removed 85 - 90% of the remaining protein.

### EXAMPLE 6 : Purification

### Continuous Mode Diafiltration

The flow through from carbon filter was further purified by using continuous mode diafiltration process. Diluted HA solution was pumped (Flow rate 15ml-20ml /min) in to cross flow filter holder equipped with 50 kDa cut off polyether sulphone cassette. (Sartocon slice cassette mod. No. 3051465001E-SG). At the beginning of the feeding of the aqueous broth in to filter, the permeate valve was closed in order to recirculate the aqueous broth for several times until the system was stable and no bubbles seen in retentate. The permeate valve was opened after 10 min and the aqueous broth was recirculated in the feed reservoir for additional 10 min to ensure no leakage of HA occurs across the membrane through the permeate valve. After 15 min. permeate and retentate were collected separately. Five equivalent volumes of sterile pyrogen free distilled water were added continuously in the reservoir containing HA solution. This addition of water to HA solution was done at same flow rate as the outflow rate of the filtrate. The inlet pressure of the cross flow filter holder was maintained around 0.5-1.5 bar. The retentate was used for further recovery of hyaluronic acid and permeate was discarded. Essentially, the permeate contained no HA. The retentate was concentrated to original volume and analyzed for purity. The diafiltration step removes 80-85 % of the remaining protein.

### Aseptic filtration

The concentrated hyaluronic acid solution from diafiltration process is finally subjected to 0.22 µm filtration aseptically. (Millipore stericup: SCGPU02RE , PVDF filter material) which renders the product that qualifies for biomedical application. The product thus formed was lyophilized to obtain medical grade high molecular weight sodium hyaluronate.

### EXAMPLE 7: Optimized Parameters of Preparation and Purification of HA

*Streptococcus zooepidemicus* ATCC 39920 was cultured in a medium composed of I % yeast extract, 1% casein hydrolyzate enzyme, 0.2% K₂HPO₄, 0.15% NaCl, 0.04% MgSO₄.7H₂O, and 5% sucrose. The organism was grown in 1 L medium at 37°C at 400 rpm for 24 h at 1 vvm aeration. The pH of the medium was maintained at 7.0 by continuous addition of NaOH aqueous solution. After the incubation, the broth was diluted with 1 volume of water and clarified by centrifugation at 10,000 rpm for 20 min at 4°C. The HA in the clarified broth was precipitated with equal volume of isopropanol. The precipitate separated by centrifugation at 10,000 rpm for 20 min at 4°C, is suspended in 1 L of 3 % sodium acetate using a mechanical homogenizer (Kinematica- A.G., polytron P T 2100) at 15000 rpm for 10 min X 3 cycles. The homogenized solution is treated with silica gel at 2 % final concentration for adsorption of protein. This step removes 85 % protein. The silica gel is separated from the sodium hyaluronate solution by centrifugation at 12,000 rpm for 20 min at 4°C. Further treatment of high molecular weight hyaluronic acid solution was done with charcoal adsorbed 0.45u filter assembly (Millipore, Millistak +, Activated Carbon minicapsule M40AC23HH3). The flow rate was 14ml/ min. This step removed 60 % of the remaining protein.

The flow through from carbon filter was further purified by using continuous mode diafiltration process. Diluted HA solution was pumped (Flow rate 15ml-20ml/min) in to cross flow filter holder equipped with 50 kDa cut off polyether sulphone cassette. (Sartocon slice cassette mod. No. 3051465001E-SG). At the beginning of the feeding of the aqueous broth in to filter, the permeate valve was closed in order to recirculate the aqueous broth for several times until the system was stable and no bubbles seen in retentate. The permeate valve was opened after 10 min and the aqueous broth was recirculated in the feed reservoir for additional 10 min to ensure no leakage of HA occurs across the membrane through the permeate valve. After 15 min. permeate and retentate were collected separately. Five equivalent volumes of sterile pyrogen free distilled water were added continuously in the reservoir containing HA solution. This addition of water to HA solution was done at same flow rate as the outflow rate of the filtrate. The inlet pressure of the cross flow filter holder was maintained around 0.5-1.5 bar. The retentate was used for further recovery of hyaluronic acid and permeate was discarded. Essentially, the permeate contained no HA. The retentate was concentrated to original volume and analyzed for purity. The diafiltration step removes 70 % of the remaining protein.

The concentrated hyaluronic acid solution from diafiltration process is finally subjected to 0.22 µm filtration aseptically. (Millipore stericup: SCGPU02RE , PVDF filter material) which renders the product that qualifies for biomedical application. The product thus formed was lyophilized to obtain high molecular wt. medical grade sodium hyaluronate with a residual protein content of 0.031 % with respect to HA with a recovery of 51 %.

### EXAMPLE 8

The process in Example 1 was scaled up to 10 L. Recovery of HA and purification profile could be reproduced. The final product resulted in a product that had 0.066 % protein with respect to HA and the recovery of HA was 82 %.

### EXAMPLE 9: Relative Analysis

The following table indicates the relative analysis:

| **S.No** | **HA purified / treated with** | **Volume / ml** | **HA yield mg/ml** | **Protein mg/ml** | **Total HA mg** | **Total Protein mg** | **% Protein w.r.t. HA** |
|---|---|---|---|---|---|---|---|
| **1** | **IPA** | **100** | **3.376** | **0.56** | **337.6** | **56** | **14.22** |
| **2** | **Silica gel** | **90** | **3.22** | **0.15** | **289.8** | **13.5** | **4.45** |
| **3** | **Carbon** | **90** | **3.114** | **0.0187** | **280.26** | **1.68** | **0.6** |
| **4** | **Dia-filtration (5X)** | **128** | **1.716** | **0.0014** | **219.6** | **0.179** | **0.075** |
| **5** | **0.22 µm filtration** | **128** | **1.96** | **0.0011** | **250.88** | **0.14** | **0.056** |

"IPA" refers to isopropanol; "S. No" refers to step number.

### EXAMPLE 10: Product Specification

The final product was tested for its protein, nucleic acid, appearance, pH, glucuronic acid content, molecular weight, IR spectra, chloride content, and moisture content. HA having a molecular weight of 3.0-3.5 million Daltons was produced. The tests mentioned above and the material specifications are as set forth in British Pharmacopoeia 2003. Comparative results are tabulated below:

| **SR.NO** | **TEST** | **SPECIFICATION** | **RESULT** |
|---|---|---|---|
| 1. | Appearance of solution | Solution clear; absorbance of solution measured at 600 nm is not greater than 0.01 | Solution clear; of solution absorbance of soution measured at 600 nm is 0.004 |
| 2. | IR spectrophotometry | The spectrum of the test substance corresponds to the reference spectrum of sodium hyaluronate | Complies |
| 3. | pH | In the range 5.0 - 8.5 | 6.65 |
| 4. | Nucleic acids | Absorbance at 260 nm does not exceed 0.5 | 0.033 |
| 5 | Protein | Not more than 0.3%. If intended for use in parenteral preparations, not more than 0.1 % | 0.056 % |
| 6. | Chlorides | 0.5 % (max) | Complies |
| 7. | Loss on Drying | Not more than 20% by weight | 18.2 % |
| 8. | Assay | Not less than 95.0% and not more than 105.0% of sodium hyaluronate calculated with reference to the dried substance | 99.2 % |

### Molecular weight determination

Molecular weight of HA was determined by size exclusion chromatography on Shodex OH Pak SB 804-805HQ column by HPLC. The mobile phase used was 0.1 M NaNO3 at a flow rate of 1ml/min using a RI detector. The molecular weight was 3.9 x10⁶ Da in 24 h All of the compositions and methods disclosed and claimed herein can be made and executed without undue experimentation in light of the present disclosure. While the methods of this invention have been described in terms of preferred embodiments, it will be apparent to those of skill in the art that variations may be applied to the methods and in the steps or in the sequence of steps of the methods described herein. More specifically, it will be apparent that certain agents that are chemically or physiologically related may be substituted for the agents described herein while the same or similar results would be achieved.

## Claims

1. A process for purifying hyaluronic acid (HA) or its salt from a bacterial fermentation broth comprising:
(a) diluting and clarifying the fermentation broth;
(b) precipitating the HA present in the broth with an equal volume of solvent;
(c) dissolving the precipitated HA or its salt in a solution;
(d) adding silica gel to the HA solution or HA salt solution from step (c) and then removing the silica gel;
(e) treating the HA solution or HA salt solution from step (d) with active carbon; and
(f) diafiltrating the HA solution or HA salt solution from step (e) using about 5 volumes of solvent;
wherein the process is conducted in the absence of any detergent or surfactant or formalin, and at neutral pH.

2. The process of claim 1, wherein the HA precipitated in step (b) is converted to its salt, and then homogenized in the solution of step (c).

3. The process of claim 2, wherein one of (i), (ii), (iii) and (iv) applies:
(i) the solution in step (c) is 3% sodium acetate;
(ii) the silica gel is removed by centrifugation in step (d);
(ii) the active carbon is impregnated on cellulose cartridge in step (e);
(iv) the solvent in step (f) is sterile pyrogen-free water.

4. The process according to claim 1, wherein the solvent used in step (b) is selected from isopropanol, ethanol and acetone.

5. The process according to claim 4, wherein the solvent is isopropanol.

6. The process of claim 1, further comprising:
(g) isolating sterile, purified HA or its salt by filtering through an aseptic filter; and
(h) lyophilizing the HA or its salt until the moisture content is less than 5%.

7. The process according to claim 1, wherein the solvent of step (b) is isopropanol, and the method further comprises (g) lyophilizing the HA or its salt until the moisture content is less than 5%.

8. A process according to any one of claims 1 to 7, wherein the fermentation broth is obtained by a process which comprises:
(i) providing a *Streptococcus* bacteria capable of producing HA;
(ii) providing a nutrient medium for culturing the bacteria, wherein the nutrient medium comprises a divalent zinc ion salt, 1% casein hydrolyzate, and 5% of a carbon source selected from the group consisting of lactose and sucrose;
(iii) culturing the bacteria in the nutrient medium; and
(iv) fermenting the nutrient medium under aerobic conditions,
wherein the hyaluronic acid has a molecular weight of at least 1 million Da.

9. The process of claim 8, wherein:
(a) the fermenting is continuous;
(b) the divalent zinc ion salt is ZnSO₄;
(c) the fermenting step is conducted for at least 15 hours at a temperature of 30-40 °C, an agitation speed of 300-500 rpm, an aeration of 1-3 vvm, and at neutral pH;
(d) the HA has a molecular weight in the range of about 3.5x10⁶ Da to 4.0x10⁶ Da, and the process produces a yield of at least 5 g of high molecular weight HA per liter of nutrient medium; or
(e) the bacteria is selected from the group consisting of *Streptococcus zooepidemicus, Streptococcus equi* and *Streptococcus pyogenes.*

10. The process of claim 9, wherein the bacteria is *Streptococcus zooepidemicus.*

11. A process according to claim 1, for producing a hyaluronic acid (HA) or its salt comprising:
(a) providing a *Streptococcus* bacteria capable of producing HA;
(b) providing a nutrient medium for culturing the microbial source, wherein the nutrient medium comprises a divalent zinc ion salt, 1% casein hydrolyzate, and 5% of a carbon source selected from the group consisting of lactose and sucrose;
(c) culturing the bacteria in the nutrient medium;
(d) fermenting the nutrient medium under aerobic conditions;
(e) diluting and clarifying the nutrient medium;
(f) precipitating the HA present in the diluted medium with a solvent;
(g) dissolving the precipitated HA or its salt in a solution;
(h) adding silica gel to the HA solution or HA salt solution from step (g) and then removing the silica gel;
(i) treating the HA solution or HA salt solution from step (h) with active carbon; and
(j) diafiltrating the HA solution or HA salt solution from step (i) using a solvent;
wherein the process is conducted in the absence of any detergent or surfactant or formalin, and at neutral pH;
wherein the HA has a molecular weight of at least 1 million Da.

## Patentansprüche

1. Verfahren zur Aufreinigung von Hyaluronsäure (HA) oder ihres Salzes aus einer bakteriellen Fermentationsbrühe, welches umfasst, dass
(a) die Fermentationsbrühe verdünnt und geklärt wird;
(b) die HA, welche in der Brühe vorhanden ist, mit einem gleichen Volumen an Lösungsmittel gefällt wird;
(c) die gefällte HA oder ihr Salz in einer Lösung gelöst wird;
(d) Kieselgel zu der HA-Lösung oder HA-Salzlösung aus Schritt (c) hinzugefügt wird und dass das Kieselgel dann entfernt wird;
(e) dass die HA-Lösung oder HA-Salzlösung aus Schritt (d) mit Aktivkohle behandelt wird; und dass
(f) die HA-Lösung oder HA-Salzlösung aus Schritt (e) diafiltriert wird, indem 5 Volumina des Lösungsmittels verwendet werden;
wobei das Verfahren in Abwesenheit jeglichen Detergenz oder Surfactants oder Formalins, und bei neutralem pH durchgeführt wird.

2. Verfahren nach Anspruch 1, wobei die HA, welche in Schritt (b) gefällt wird, in ihr Salz umgewandelt wird, und dann in der Lösung aus Schritt (c) homogenisiert wird.

3. Verfahren nach Anspruch 2, wobei eines aus (i), (ii), (iii) und (iv) zutrifft:
(i) die Lösung in Schritt (c) ist 3%iges Natriumacetat;
(ii) das Kieselgel wird durch Zentrifugation in Schritt (d) entfernt;
(iii) die Aktivkohle in Schritt (e) ist auf eine Cellulosekartusche geprägt;
(iv) das Lösungsmittel in Schritt (f) ist steriles, pyrogenfreies Wasser.

4. Verfahren nach Anspruch 1, wobei das Lösungsmittel, welches in Schritt (b) verwendet wird, aus Isopropanol, Ethanol und Aceton ausgewählt wird.

5. Verfahren nach Anspruch 4, wobei das Lösungsmittel Isopropanol ist.

6. Verfahren nach Anspruch 1, welches darüber hinaus umfasst, dass
(g) sterile aufgereinigte HA oder ihr Salz durch Filtern durch einen aseptischen Filter isoliert wird; und dass
(h) die HA oder ihr Salz lyophilisiert wird, bis der Feuchtigkeitsgehalt weniger als 5% ist.

7. Verfahren nach Anspruch 1, wobei das Lösungsmittel in Schritt (b) Isopropanol ist, und das Verfahren darüber hinaus umfasst, dass (g) die HA oder ihr Salz lyophilisiert wird, bis der Feuchtigkeitsgehalt weniger als 5% ist.

8. Verfahren nach einem der Ansprüche 1 bis 7, wobei die Fermentationsbrühe durch ein Verfahren erhalten wird, welches umfasst, dass
(i) ein Streptococcus-Bakterium, welches fähig ist, HA herzustellen, zur Verfügung gestellt wird;
(ii) dass ein Nährmedium zur Bakterienzucht zur Verfügung gestellt wird, wobei das Nährmedium ein zweiwertiges Zinkionensalz, 1%iges Caseinhydrolysat und 5% einer Kohlenstoffquelle, welche aus der Gruppe, welche aus Lactose und Saccharose besteht, ausgewählt ist, aufweist;
(iii) das Bakterium in dem Nährmedium gezüchtet wird, und
(iv) das Nährmedium unter aeroben Bedingungen fermentiert wird, wobei die Hyaluronsäure ein Molekulargewicht von mindestens 1 Mio. Da hat.

9. Verfahren nach Anspruch 8, wobei
(a) die Fermentation kontinuierlich ist;
(b) das zweiwertige Zinkionensalz ZnSO₄ ist;
(c) der Fermentationsschritt mindestens 15 Stunden lang bei einer Temperatur von 30 bis 40°C, einer Rührgeschwindigkeit von 300 bis 500 U/min, einer Belüftungsrate von 1 bis 3 vvm, und einem neutralen pH durchgeführt wird;
(d) die HA ein Molekulargewicht im Bereich von etwa 3,5 x 10⁶ Da bis 4,0 x 10⁶ Da hat, und das Verfahren eine Ausbeute von mindestens 5 g einer hochmolekulargewichtigen HA/I Nährmedium erzeugt; oder
(e) das Bakterium aus der Gruppe ausgewählt ist, welche aus *Streptococcus zooepidemicus, Streptococcus equi* und *Streptococcus pyogenes* besteht.

10. Verfahren nach Anspruch 9, wobei das Bakterium *Streptococcus zooepidemicus* ist.

11. Verfahren nach Anspruch 1, zur Herstellung einer Hyaluronsäure (HA) oder ihres Salzes, welches umfasst, dass
(a) ein Streptococcus-Bakterium, welches fähig ist, HA herzustellen, zur Verfügung gestellt wird;
(b) ein Nährmedium zur Zucht der mikrobiellen Quelle zur Verfügung gestellt wird, wobei das Nährmedium ein zweiwertiges Zinkionensalz, 1%iges Caseinhydrolysat und 5% einer Kohlenstoffquelle, welche aus der Gruppe ausgewählt ist, welche aus Lactose und Saccharose besteht, aufweist;
(c) das Bakterium in dem Nährmedium gezüchtet wird;
(d) das Nährmedium unter aeroben Bedingungen fermentiert wird;
(e) das Nährmedium verdünnt und geklärt wird;
(f) die HA, welche in dem verdünnten Medium vorhanden ist, mit einem Lösungsmittel gefällt wird;
(g) die gefällte HA oder ihr Salz in einer Lösung gelöst wird;
(h) Kieselgel zu der HA-Lösung oder HA-Salzlösung aus Schritt (g) hinzugefügt wird und das Kieselgel dann entfernt wird;
(i) die HA-Lösung oder HA-Salzlösung aus Schritt (h) mit Aktivkohle behandelt wird; und
(j) die HA-Lösung oder HA-Salzlösung aus Schritt (i) mittels eines Lösungsmittels diafiltriert wird;
wobei das Verfahren in Abwesenheit jeglichen Detergenz oder Surfactant oder Formalin, und bei neutralem pH durchgeführt wird;
wobei die HA ein Molekulargewicht von mindestens 1 Mio. Da hat.

## Revendications

1. Procédé pour purifier l'acide hyaluronique (HA) ou son sel à partir d'un bouillon de fermentation bactérienne comprenant :
(a) la dilution et la clarification du bouillon de fermentation ;
(b) la précipitation du HA présent dans le bouillon avec un même volume de solvant ;
(c) la dissolution du HA précipité ou de son sel dans une solution ;
(d) l'addition de gel de silice à la solution de HA ou à la solution de sel de HA provenant de l'étape (c) puis le retrait du gel de silice ;
(e) le traitement de la solution de HA ou de la solution de sel de HA provenant de l'étape (d) avec du carbone activé ; et
(f) la diafiltration de la solution de HA ou de la solution de sel de HA provenant de l'étape (e) au moyen d'environ 5 volumes de solvant ;
où le procédé est conduit en l'absence de tout détergent ou tensioactif ou formol, et à pH neutre.

2. Procédé selon la revendication 1, où le HA précipité dans l'étape (b) est converti en son sel, puis homogénéisé dans la solution de l'étape (c).

3. Procédé selon la revendication 2, où l'un de (i), (ii), (iii) et (iv) s'applique :
(i) la solution dans l'étape (c) est de l'acétate de sodium à 3%;
(ii) le gel de silice est retiré par centrifugation dans l'étape (d) ;
(ii) le carbone activé imprègne une cartouche de cellulose dans l'étape (e) ;
(iv) le solvant dans l'étape (f) est de l'eau sans pyrogènes stérile.

4. Procédé selon la revendication 1, où le solvant utilisé dans l'étape (b) est choisi parmi l'isopropanol, l'éthanol et l'acétone.

5. Procédé selon la revendication 4, où le solvant est l'isopropanol.

6. Procédé selon la revendication 1, comprenant en outre :
(g) l'isolement du HA purifié stérile ou de son sel par filtration sur un filtre aseptique ; et
(h) la lyophilisation du HA ou de son sel jusqu'à ce que la teneur en humidité soit inférieure à 5 %.

7. Procédé selon la revendication 1, où le solvant de l'étape (b) est l'isopropanol, et le procédé comprend en outre (g) la lyophilisation du HA ou de son sel jusqu'à ce que la teneur en humidité soit inférieure à 5 %.

8. Procédé selon l'une quelconque des revendications 1 à 7, où le bouillon de fermentation est obtenu par un procédé qui comprend :
(i) la fourniture d'une bactérie *Streptococcus* capable de produire HA ;
(ii) la fourniture d'un milieu nutritif pour cultiver la bactérie, où le milieu nutritif comprend un sel à ion zinc divalent, un hydrolysat de caséine à 1 %, et 5 % d'une source de carbone choisie dans le groupe consistant en le lactose et le saccharose ;
(iii) la culture de la bactérie dans le milieu nutritif ; et
(iv) la fermentation du milieu nutritif dans les conditions aérobies,
où l'acide hyaluronique a une masse moléculaire d'au moins 1 million de Da.

9. Procédé selon la revendication 8, où :
(a) la fermentation est continue ;
(b) le sel à ion zinc divalent est ZnSO₄ ;
(c) l'étape de fermentation est conduite pendant au moins 15 heures à une température de 30-40°C, une vitesse d'agitation de 300-500 tr/min, une aération de 1,3 vvm, et à pH neutre ;
(d) le HA a une masse moléculaire dans la plage d'environ 3,5x10⁶ Da à 4,0x10⁶ Da, et le procédé produit un rendement d'au moins 5 g de HA de haute masse moléculaire par litre de milieu nutritif ; ou
(e) la bactérie est choisie dans le groupe consistant en *Streptococcus zooepidemicus, Streptococcus equi* et *Streptococcus pyogenes.*

10. Procédé selon la revendication 9, où la bactérie est *Streptococcus zooepidemicus.*

11. Procédé selon la revendication 1, pour produire un acide hyaluronique (HA) ou son sel comprenant :
(a) la fourniture d'une bactérie *Streptococcus* capable de produire HA ;
(b) la fourniture d'un milieu nutritif pour cultiver la source microbienne,
où le milieu nutritif comprend un sel à ion zinc divalent, un hydrolysat de caséine à 1 %, et 5 % d'une source de carbone choisie dans le groupe consistant en le lactose et le saccharose ;
(c) la culture de la bactérie dans le milieu nutritif ;
(d) la fermentation du milieu nutritif dans des conditions aérobies ;
(e) la dilution et la clarification du milieu nutritif ;
(f) la précipitation du HA présent dans le milieu dilué avec un solvant ;
(g) la dissolution du HA précipité ou de son sel dans une solution ;
(h) l'addition de gel de silice à la solution de HA ou à la solution de sel de HA provenant de l'étape (g) puis le retrait du gel de silice ;
(i) le traitement de la solution de HA ou de la solution de sel de HA provenant de l'étape (h) avec du carbone activé ; et
(j) la diafiltration de la solution de HA ou de la solution de sel de HA provenant de l'étape (i) au moyen d'un solvant ;
où le procédé est conduit en l'absence de tout détergent ou tensioactif ou formol, et à pH neutre ;
où le HA a une masse moléculaire d'au moins 1 million de Da.
